# EUROPÄISCHE PATENTANMELDUNG

(11) **EP 3 865 583 A1**
(43) Veröffentlichungstag der Anmeldung: **18.08.2021**
(21) Anmeldenummer: 21157058.5
(22) Anmeldetag: 15.02.2021
(51) Int. Cl.: C12Q 1/04, G01N 33/569

(54) **VERFAHREN ZUM NACHWEIS VON MIKROORGANISMEN UND SCHEIBENFÖRMIGER PROBENTRÄGER**

(30) Priorität: 14.02.2020 DE 102020103963; 11.02.2021 US 202117173488
(71) Anmelder: Testo bioAnalytics GmbH, 79822 Titisee-Neustadt (DE)
(72) Erfinder: Riemer, Joel, 79874 Breitnau (DE)
(74) Vertreter: Mertzlufft-Paufler, Cornelius

(57) **Zusammenfassung**

Die Erfindung betrifft ein Nachweisverfahren (1) für antibiotikaresistente Mikroorganismen (2), wobei dabei wenigstens eine spezifische Substanz (3), die durch ein eine Antibiotikaresistenz bewirkendes Enzym (8) des Mikroorganismus (2) zersetzt wird, der Probe (5) zugegeben wird, wobei durch das vorhandene Enzym (8) eine Reaktion (4) ausgelöst wird, wobei bei der Reaktion (4) ein optisch nachweisbares Reaktionsprodukt (6) in der Nähe zum resistenten Mikroorganismus (2) erzeugt wird, das anschließend in einem optischen Nachweisverfahren detektiert (7) wird (Fig. 1).

## Beschreibung

Die Erfindung betrifft ein Verfahren zum Nachweis von Mikroorganismen und/oder derer Eigenschaften, wobei wenigstens eine spezifische Substanz, die eine Reaktion des nachzuweisenden Mikroorganismus auslöst, einer Probe, in der die Mikroorganismen nachgewiesen werden sollen, beigegeben wird, wobei bei der Reaktion ein optisch nachweisbares Reaktionsprodukt erzeugt wird, das anschließend in einem optischen Nachweisverfahren detektiert wird.

Es sind bereits unterschiedliche Verfahren eingangs genannter Art bekannt, die zum Beispiel zum Nachweis von antibiotikaresistenten Bakterien insbesondere in Krankenhäusern, beispielsweise in Human-Diagnostik, Anwendung finden.

Man kennt dabei beispielsweise kultivierungsbasierte Verfahren. Diese weisen jedoch den Nachteil auf, dass sie einen hohen Zeitaufwand haben, bis das Ergebnis des Tests feststeht, da sie auf viele notwendige Zellteilungsschritte der Mikroorganismen angewiesen sind. Zudem ist das Vornehmen von Korrekturmaßnahmen, wie zielgerichteten Gegenmaßnahmen gegen die Mikroorganismen wie beispielsweise Gabe vom korrekten Antibiotikum, während der Durchführung des Verfahrens, d.h. bis das Ergebnis verfügbar ist, nicht möglich.

Des Weiteren kennt man bereits Schnellmessverfahren, die darauf basieren, dass auf DNA oder RNA zum Nachweis bestimmter Eigenschaften (wie z.B. Antibiotikaresistenz) zurückgegriffen wird. Diese Verfahren eignen sich zwar zum Nachweis der genetischen Veranlagung der Mikroorganismen, wenn z.B. ein Resistenzgen nachgewiesen wird. Allerdings bieten sie keinen Nachweis der tatsächlichen phänotypischen Ausprägung einer Eigenschaft, da der Nachweis nicht auf Proteinebene, also über die phänotypische Ausprägung bzw. das tatsächliche Vorhandensein beispielsweise von Resistenzproteinen, erfolgt. Darüber hinaus haben Nukleinsäure-basierten Nachweisverfahren den Nachteil, dass bei auftretenden Mutationen in den Zielsequenzen der DNA/RNA die Zielsequenzen nicht mehr erkannt werden, eventuell aber dennoch eine Resistenz vorliegt und so ein falsch-negatives Ergebnis erzeugt wird. Andere phänotypische Nachweisverfahren weisen wiederum den Nachteil auf, dass sie für die Bestimmung eine hohe Mikroorganismenanzahl benötigen, um ein ausreichend hohes Signal zu erzeugen, welches detektiert werden kann, wie beispielsweise Farbumschläge um Mikroorganismenkolonien auf Agarplatten. Somit wird an dieser Stelle wieder viel Zeit bis zum Abschluss einer Analyse benötigt.

Andere Schnellmessmethoden sind lediglich indikativ und nicht quantitativ. Zudem bieten sie keine Möglichkeit der Unterscheidung zwischen lebenden und toten Mikroorganismen.

Es besteht somit die Aufgabe, ein verbessertes Verfahren zum Nachweis von Mikroorganismen eingangs genannter Art zu schaffen, bei welchem die Nachteile vorbekannter Verfahren ausgeräumt sind.

Die Lösung dieser Aufgabe wird erfindungsgemäß durch ein Verfahren mit den Merkmalen nach Anspruch 1 bereitgestellt.

Insbesondere wird erfindungsgemäß zur Lösung der Aufgabe ein Verfahren eingangs genannter Art vorgeschlagen, bei welchem vorgesehen ist, dass das Reaktionsprodukt bis zum optischen Nachweis in räumlicher Nähe zum reagierenden Mikroorganismus gehalten wird. Das Verfahren bietet die Vorteile, dass eine sehr niedrige Nachweisgrenze dadurch erreichbar ist, dass das Vorhandensein einzelner Mikroorganismen nachgewiesen wird, indem eine Anreicherung des Reaktionsproduktes in der Nähe des Mikroorganismus erfolgt, so dass ein stärkeres Signal entsteht, das leichter nachweisbar ist, als bei bisher vorhandenen Verfahren. Somit ist das Verfahren außerdem sicherer als Verfahren, die auf eine Kultivierung und Vermehrung der Mikroorganismen angewiesen sind. Die Durchführung kann somit in-house, also ohne Labor, on-site und ohne eine labortechnische Ausbildung erfolgen, da keine Mikroorganismen angereichert werden müssen. Somit besteht keine Kontaminationsgefahr für Mensch und Umwelt.

Zudem ist eine absolute Quantifizierung der positiven Mikroorganismen möglich, da kein RNA- und/oder DNA-Nachweis vorgenommen wird, sondern ein Nachweis auf Proteinebene erfolgt und dieser beispielsweise auch Einzelorganismen zugeordnet werden kann. Somit kann eine echte Aussage über das Vorhandensein eines bestimmten Phänotyps eines Mikroorganismus in einer Probe getroffen werden. Das Verfahren weist weiter eine gegenüber vorbekannten Verfahren kürzere Analysezeit auf, so dass betroffene Patienten ggfls. schneller behandelt werden können. Aufgrund der leichten Ausführbarkeit und der schnellen Analysezeit des Verfahrens eignet es sich besonders dazu, um ohne Labor und vor-Ort ausgeführt zu werden. So kann es in Krankenhäusern, insbesondere sogar auf Krankenhausstationen, Anwendung finden. Das Verfahren ist zudem kostengünstiger als laborbasierte Verfahren, da auf Equipment und Ausführungsschritte verzichtet werden kann. Das System kann für eine Detektion von Antibiotikaresistenzen verwendet werden, kann aber ebenso auf die Detektion von anderen Eigenschaften von Mikroorganismen verwendet werden.

Im Falle des Nachweises eines Bakteriums kann die spezifische Substanz beispielsweise chemisch an eine Zellwand und/oder eine Hülle des Bakteriums gebunden werden.

Nachfolgend werden vorteilhafte Ausgestaltungen der Erfindung beschrieben, die allein oder in Kombination mit den Merkmalen anderer Ausgestaltungen optional zusammen mit den Merkmalen nach Anspruch 1 kombiniert werden können.

Ergänzend zur optischen Auswertung können auch biochemische Verfahren zur Detektion verwendet werden.

Gemäß einer vorteilhaften Weiterbildung kann es vorgesehen sein, dass das Reaktionsprodukt an eine Außenfläche des Mikroorganismus anbindet. Somit kann besonders gut gewährleistet werden, dass es zu keiner Trennung des Reaktionsproduktes vom Mikroorganismus kommt, was beispielsweise dazu führen könnte, dass ein fehlerhaftes Analyseergebnis entsteht. Beispielsweise kann sich dadurch ein Reaktionsprodukt in der Nähe oder innerhalb eines Mikroorganismus besser lokal anreichern, bis ein Nachweisschwellenwert erreicht ist, der zu einem positiven Nachweisergebnis führt. Durch die räumliche Konzentration ist zudem die Zeitspanne geringer, die abgewartet werden muss, um den Nachweis durchführen zu können. Zudem kann die Sensitivität des Verfahrens erhöht werden, da bereits einzelne positive Mikroorganismen erfassbar sind. Der Vorteil der räumlichen Konzentrierung ist, dass eine detektierbare Konzentration eines Reaktionsproduktes schneller erreicht werden kann, als wenn das Produkt in einem größeren Volumen (beispielsweise in einer größeren Reaktionskammer) verteilt wird. Die Reaktion kann somit schneller durchgeführt werden, wobei gleichzeitig weniger Hintergrundrauschen entsteht.

Beispielsweise kann die Anbindung des Reaktionsprodukts an den Mikroorganismus über einen Vermittler, wie z.B. einen Antikörper, erfolgen, an welchem die spezifische Substanz gebunden ist. Durch Bindung des spezifischen Antikörpers an ein Antigen des Mikroorganismus, kann die spezifische Substanz und/oder das Reaktionsprodukt lokal an der Außenseite des Mikroorganismus festgehalten und/oder aufkonzentriert werden. Zudem kann das Reagenz auch so gestaltet werden, dass das resultierende Reaktionsprodukt hochreaktiv ist und unmittelbar mit seiner Umgebung (beispielsweise mit der Hülle der Mikroorganismen) reagiert und so räumlich in der Nähe des Reaktionsortes fixiert wird.

Gemäß einer weiteren vorteilhaften Weiterbildung kann es vorgesehen sein, dass die einzelnen Mikroorganismen von einer Phasengrenze zu einem außen liegenden Trägerfluid umgeben werden. Insbesondere kann als Trägerfluid eine vorzugsweise hydrophobe Flüssigkeit, wie Öl, oder Luft verwendet werden. Insbesondere kann das Reaktionsprodukt durch die Phasengrenzen eingeschlossen werden. Bei dieser Ausgestaltung handelt es sich um eine ergänzende und/oder alternative Ausgestaltung zu der im vorhergehenden Absatz beschriebenen Ausgestaltung. Die Vorteile sind dabei die gleichen, da auch hierbei eine bessere lokale Anreicherung des Reaktionsproduktes in der Nähe des Mikroorganismus und ein Verhindern einer Dissoziation des Reaktionsproduktes ermöglicht ist. Die erwähnte Phasengrenze kann beispielsweise dadurch erreicht werden, dass die mit der Substanz (Substrat) versetzten Mikroorganismen durch eine Düse in eine Trägerflüssigkeit und/oder ein Trägerfluid eingebracht werden, wobei die Düse als Zerstäuberdüse ausgebildet sein kann. Besonders zweckmäßig kann es dabei sein, wenn die Mikroorganismen, insbesondere zusammen mit der spezifischen Substanz, in Tröpfchen (Droplets) eingebracht werden. Die Mikroorganismen können zum Beispiel in einem wässrigen Milieu gehalten werden.

Gemäß einer vorteilhaften Ausgestaltung kann es vorgesehen sein, dass ein empfindlicher Bereich des Nachweisverfahrens auf eine Abmessung der Mikroorganismen eingestellt ist. Somit ist eine Einzelerfassung von Mikroorganismen einfach durchführbar, beispielsweise für ein Zählen.

Um insbesondere einheitlich große Tröpfchen ausbilden zu können, in welchen ein Mikroorganismus oder mehrere Mikroorganismen eingeschlossen ist/sind, kann es vorgesehen sein, dass eine die einzelnen Mikroorganismen umgebende Phasengrenze, beispielsweise die bereits zuvor genannte Phasengrenze, durch Einsprühen der Mikroorganismen in ein Trägerfluid, beispielsweise das bereits zuvor genannte Trägerfluid, erzeugt wird.

Gemäß einer weiteren vorteilhaften Ausgestaltung kann es vorgesehen sein, dass das Reaktionsprodukt auf einen Raum von weniger als 1000 µm Durchmesser beschränkt wird. Da keine Kultivierung der Mikroorganismen vor Durchführung des Verfahrens erforderlich ist, kann der Materialbedarf für die Durchführung des Verfahrens deutlich reduziert werden. Dies ist zudem insofern vorteilhaft, da die erforderlichen Substanzen häufig gesundheitsschädlich oder zumindest gesundheitlich bedenklich sind.

Gemäß einer weiteren vorteilhaften Ausgestaltung kann es vorgesehen sein, dass die untersuchten Mikroorganismen auf einem Filtermaterial zurückgehalten werden (z.B. einer track etched Membran) und die Untersuchung auf dieser abläuft und/oder zusätzlich diese für die Konzentrierung bzw. Abscheidung der Organismen aus einem größeren Probenvolumina verwendet wird. Dadurch können größere Probenvolumina eingebracht werden und eingesetzte Reagenzien durch das geringe Probenvolumina eingespart bzw. genauer konzentriert verwendet werden.

Um die Durchführung des Verfahrens zu beschleunigen, kann es gemäß einer weiteren Ausgestaltung vorgesehen sein, dass die Probe nach Beigabe der wenigstens einen Substanz erwärmt wird. Insbesondere kann die Probe dabei um 10°C, vorzugsweise um mindestens 10°C oder mehr, erwärmt werden. Grundsätzlich kann jedoch gesagt werden, dass das beschriebene Verfahren gegenüber vorbekannten Nachweisverfahren bereits deutlich schneller, auch ohne Erwärmung, durchführbar ist. Eine klassische kultivierungsbasierte Methode, wie die Kultivierung auf Nährböden/Agarplatten und Verwendung von Abklatschplatten, benötigt bereits gleich mehrere Tage, da hierbei stets eine Kultivierung erforderlich ist, die beim beanspruchten Verfahren entfallen kann.

Alternativ oder ergänzend dazu kann es gemäß einer weiteren vorteilhaften Ausgestaltung vorgesehen sein, dass das Reaktionsprodukt in einer nährmittelfreien Umgebung und/oder kultivierungsfrei erzeugt wird. Somit kann gewährleistet werden, dass es zu keiner ungewollten Vermehrung von Mikroorganismen während der Durchführung des Verfahrens kommt.

Gemäß einer weiteren vorteilhaften Ausgestaltung kann es vorgesehen sein, dass als wenigstens eine spezifische Substanz eine profluoreszierende und/oder eine prolumineszierende und/oder eine prophosphoreszierende Substanz und/oder eine Substanz, die einen Farbumschlag bewirkt, verwendet wird. Werden diese Substanzen (Substrate) durch Proteine, wie spezifische Enzyme als Katalysatoren der Zielorganismen, umgesetzt, so können sie anschließend als Reaktionsprodukt detektiert werden. Diese Detektion kann zum Beispiel mittels eines Zytometers vorgenommen werden. Insbesondere mittels eines Zytometers eines scheibenförmigen Probenträgers (lab on a chip). Der Probenträger kann zu einer Mikrofluidik vorgesehen sein. Dabei können beispielsweise profluoreszierende Substrate verwendet werden, welche ähnliche Strukturen wie β-Lactam-Antibiotika besitzen. Sind z.B. Betalactamase-bildende gramnegative Mikroorganismen, also potentiell antibiotikaresistenten Mikroorganismen, in einer Probe vorhanden, so setzen diese das gewählte Substrat mit deren außenmembranständigen Enzymen, wie Betalactamasen und/oder Carbapenemasen, um. Das Reaktionsprodukt ist dann messbar, so dass das Ergebnis Aufschluss darüber gibt, ob die Bakterien Antibiotika abbauen können. Gerade in Krankenhäusern spielen derartige Verfahren eine wichtige Rolle, um multiresistente Keime frühzeitig erkennen zu können, um deren Ausbreitung schnellstmöglich einzudämmen. Grundsätzlich kann das erfindungsgemäße Verfahren jedoch auch zum Nachweis anderer Proteine oder Enzyme verwendet werden. Vorzugsweise kann es sich dabei um Proteine und/oder Enzyme handeln, die an einer Außenseite eines Mikroorganismus liegen.

Um einen kritischen Analyseschwellenwert festlegen zu können, kann es vorgesehen sein, dass eine optische Nachweisgrenze so gesetzt ist, dass das Reaktionsprodukt erst ab einer vorbestimmten Konzentration detektiert wird. Somit ist es möglich, die Sensitivität des Verfahrens an die jeweiligen Bedürfnisse bedarfsgerecht anzupassen.

Gemäß einer weiteren vorteilhaften Ausgestaltung kann bei dem Verfahren ein Schritt zur Bestimmung von lebenden und/oder toten Mikroorganismen vorgenommen werden. Beispielsweise kann dies durch vorbekannte Substanzen (Substrate) erfolgen, mittels welchen die lebenden und/oder toten Zellen beispielsweise eingefärbt werden können. So ist auch hier beispielsweise eine optische Auswertung möglich. Vorbekannte Verfahren, die auf der Basis von DNA-/RNA-Sonden beruhen, weisen hingegen den Nachteil auf, dass damit keine Differenzierung zwischen den lebenden und toten Zellen vorgenommen werden kann. Eine derartige Ausführungsform kann auch insbesondere in Kombination mit einer Ausgestaltung der Messung auf dem vorgenannten Filtermaterial vorteilhaft sein, da auf eine solche Weise große Probenvolumina filtriert, auf diese Art Mikroorganismen auf dem zu untersuchenden Filtermaterial zurückgehalten und diese anschließend hinsichtlich z.B. ihrer Vitalität (lebend/tot/koloniebildend/nicht-koloniebildend) untersucht werden können. Hierzu kann es nützlich sein, eine Inkubation der Organismen mit oder ohne einem Nährmedium auf dem Filtermaterial oder in einer Kavität des fluidischen Systems durchzuführen und ein Reagenz hinzuzugeben, welches ausschließlich lebende und/oder vermehrungsfähige Organismen markiert. Somit kann eine Probe hinsichtlich ihrer Sterilität und/oder Konzentration von lebenden Mikroorganismen untersucht werden.

Um zu vermeiden, dass die Reaktionsprodukte und/oder die Mikroorganismen aus einem Messkreislauf austreten können, kann es gemäß einer Weiterbildung vorgesehen sein, dass die eingeführte und dem Nachweis unterzogene Probe anschließend biologisch deaktiviert wird, beispielsweise durch Autoklavieren.

Die Erfindung betrifft außerdem einen scheibenförmigen Probenträger mit Mitteln zum Ausführen eines Verfahrens, wie es hierin beschrieben und/oder beansprucht ist. Der Probenträger kann beispielsweise mit einer Mikrofluidik versehen sein, beispielsweise mit einem Kanalsystem mit mikrofluidischen Kanälen. Dies ermöglicht auf einfache Weise eine getrennte Verarbeitung einzelner Mikroorganismen. Insbesondere weist der Probenträger einen Aufnahmeraum zur Abnahme der Mikroorganismen von einem Probennahmeinstrument und/oder eine Düse zum Einsprühen der Mikroorganismen in ein Trägerfluid und/oder einen Nachweisbereich zu einer quantitativen optischen Detektion von Mikroorganismen, in deren räumlicher Nähe sich das Reaktionsprodukt befindet, auf. Der Probenträger weist den Vorteil auf, mit diesem einen Nachweis eines bestimmten Mikroorganismus beispielsweise vor Ort, wie im Krankenhaus, vornehmen zu können. Somit kann mittels des Probenträgers ein Schnelltest auf Proteinebene durchgeführt werden. Eine besondere labortechnische Ausbildung oder Schulung des Nutzers ist dabei nicht erforderlich. Zudem sind die zur Durchführung des Verfahrens zu treffenden Sicherheitsvorkehrungen aufgrund der fehlenden Vermehrung der Mikroorganismen zur Durchführung des Verfahrens als deutlich geringer als bei vorbekannten Verfahren einzustufen.

Ein Ziel der Erfindung kann es sein, eine spezifische und schnelle Detektion von Eigenschaften von Mikroorganismen wie z.B. das Vorhandensein von Carbapenemasen, anderen betaLactamasen und/oder einer Kombination aus mehreren Parametern mittels einer vorzugsweise handlings-freien Detektion innerhalb eines lab-on-a-chip Systems bereitzustellen. Das System soll z.B. Patienten- oder Krankenhausumgebungs-Proben automatisch aufbereiten und vermessen. Hierbei sollen beispielsweise ESBL-bildende Bakterien spezifisch markiert und anschließend gezählt werden. Wünschenswert ist dabei eine Diskriminierung zwischen lebenden und toten Mikroorganismen. Die lebend/tot-Unterscheidung kann beispielsweise an dem Vorhandensein oder Nicht-Vorhandensein von Enzymaktivität durchgeführt werden. Auch kann das Verfahren um Verfahren erweitert werden, so dass auch die Bakterienart bestimmt wird. So kann beispielsweise das Ergebnis einer Analyse sein: es liegt das Vorhandensein einer Carbapenemase vor und der Organismus "Acinetobacter baumanii" wurde erkannt. So ist eine Gefahrenbeurteilung noch deutlich besser, einfacher und schneller als bisher möglich war.

### Ausführungsbeispiele:

Die Erfindung wird nun anhand eines Ausführungsbeispiels näher beschrieben, ist jedoch nicht auf dieses Ausführungsbeispiel beschränkt. Weitere Ausführungsbeispiele ergeben sich durch die Kombination der Merkmale einzelner oder mehrerer Ansprüche untereinander und/oder mit einzelnen oder mehreren Merkmalen der Ausführungsbeispiele.

Es zeigt:
- Fig. 1: eine erste Ausführungsvariante eines erfindungsgemäßen Verfahrens in einer schematischen Darstellung,
- Fig. 2: eine weitere Ausführungsvariante eines erfindungsgemäßen Verfahrens in einer schematischen Darstellung.

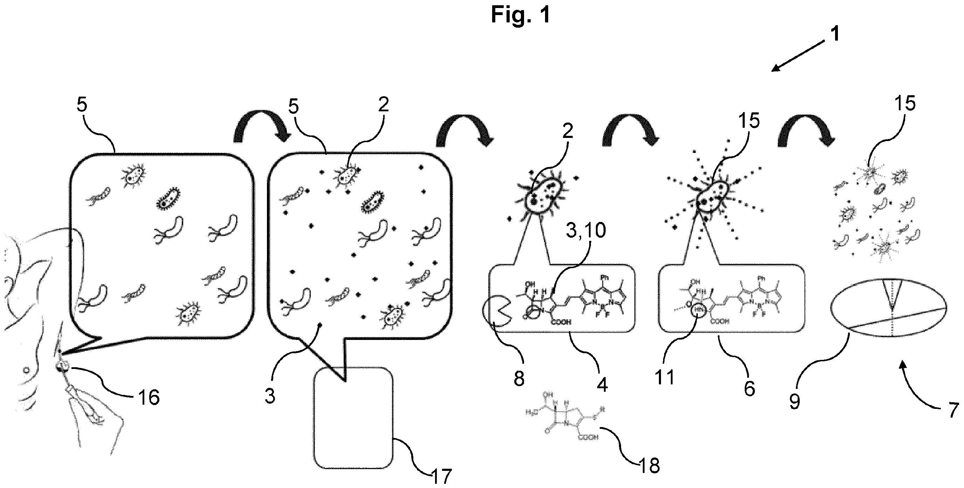

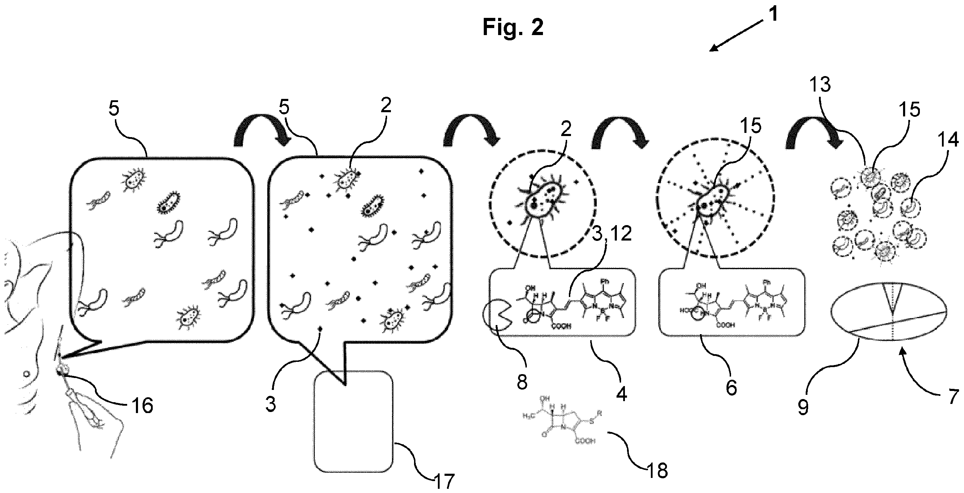

In den Figuren 1 und 2 ist ein Verfahren zum Nachweis von Mikroorganismen in verschiedenen Ausführungen gezeigt.

Fig. 1 zeigt ein erfindungsgemäßes Verfahren 1 zum Nachweis von Mikroorganismen 2, wobei wenigstens eine spezifische Substanz 3, die eine Reaktion 4 des nachzuweisenden Mikroorganismus 2 auslöst, einer Probe 5, in der die Mikroorganismen 2 nachgewiesen werden sollen, beigegeben wird. Als spezifische Substanz 3 (in diesem Fall ein spezifisches Färbereagenz) werden einer zu untersuchenden Probe 5 profluoreszierende und/oder prolumineszierende und/oder prophosphoreszierende Substrate und/oder Substrate, welche einen Farbumschlag bewirken, zugegeben. Die spezifische Substanz 3 kann spezifisch an ein bestimmtes Antigen des Mikroorganismus 2 gebunden werden. Vorzugsweise wird die spezifische Substanz 3 an eine Außenseite des intakten und/oder lebenden Mikroorganismus 2 angebunden.

Bei der Reaktion 4 wird ein optisch nachweisbares Reaktionsprodukt 6 erzeugt, das anschließend in einem optischen Nachweisverfahren detektiert 7 wird. Bis zum optischen Nachweis 7 wird das Reaktionsprodukt 6 in räumlicher Nähe zum reagierenden Mikroorganismus 2 gehalten.

Werden die zuvor genannten Substrate (spezifische Substanz 3) durch wenigstens ein spezifisches Enzym 8 als Katalysator der Mikroorganismen 2 (Zielorganismen) umgesetzt, so sind diese zum Beispiel im Zytometer 9 (Zellzähl- und Zellanalysegerät), insbesondere eines Lab-on-a-chip Systems, erkennbar.

Dabei kann es sich zum Beispiel um wenigstens ein profluoreszierendes Substrat handeln, welches ähnliche Strukturen wie β-Lactam-Antibiotika besitzt. Sind z.B. Betalactamase-bildende gramnegative Mikroorganismen, also potentiell antibiotikaresistente Mikroorganismen, in einer Probe 5 vorhanden, so setzen diese das gewählte Substrat mit deren außenmembranständigen Betalactamasen um.

Das fluoreszente Reaktionsprodukt 6 wird auf der Außenmembran der Mikroorganismen 2 fixiert, z.B. durch selbst-immobilisierende Substrate 10, welche nach deren Umsetzung (und Bildung eines radikalen/reaktiven Eduktes 11) z.B. an den Membranstrukturen/-proteinen der Mikroorganismen binden.

Fig. 2 zeigt eine weitere Ausführungsvariante eines erfindungsgemäßen Verfahrens 1 zum Nachweis von Mikroorganismen 2, wobei wenigstens eine spezifische Substanz 3, die eine Reaktion 4 des nachzuweisenden Mikroorganismus 2 auslöst, einer Probe 5, in der die Mikroorganismen 2 nachgewiesen werden sollen, beigegeben wird.

Aus Fig. 2 ist weiterhin ersichtlich, dass als spezifische Substanz 3 Substrate 12 zugegeben werden können, welche nach deren Umsetzung dissoziieren. In diesem Fall können die Mikroorganismen vor Analyse in Tröpfchen 13, 14 (Droplets) mit geringen Volumen eingehüllt werden, um das Signal lokal aufzukonzentrieren. Die Tröpfchen 13, 14 können dabei zum Beispiel ein Volumen von maximal 2000 µL, insbesondere maximal 1500 µL, insbesondere maximal 1000 µL, insbesondere maximal 750 µL, insbesondere maximal 500 µL, insbesondere maximal 400 µL, insbesondere maximal 300 µL, insbesondere maximal 200 µL, insbesondere maximal 100 µL, aufweisen.

Ebenso ist die Kombination mit anderen Färbemethoden möglich, um unterschiedliche Parameter zu detektieren, wie beispielsweise eine zusätzliche Antikörpermarkierung und/oder eine Färbung mit einem Farbstoff zur Detektion lebender und/oder toter Mikroorganismen.

Anschließend wird die Probe 5 per Zytometrie oder anderen optischen Verfahren, wie beispielsweise bildgebenden Verfahren wie Fluoreszenzmikroskopie, vermessen 7 und die Anzahl der fluoreszierenden und somit potentiell antibiotikaresistenten (insbesondere lebenden) Mikroorganismen 15 festgestellt.

Die Technologie ist ebenfalls für die Detektion anderer spezifischer Enzymaktivitäten nutzbar - insbesondere wenn die Produkte der Reaktion nicht im Zellinneren der Zielorganismen vorliegen bzw. dissoziieren.

Das zuvor genannte Beispiel bezieht sich auf ein antibiotikaähnliches Substrat zur Detektion von Carbapenemasen-bildenden Organismen.

Die Erfindung bezieht sich auf den Vorschlag der Nutzung spezifischer Substrate in einem Lab-on-a-chip System in Kombination mit einer lokalen Fixierung des Signals zur Detektion von z.B. Antibiotikaresistenz.

In einer bevorzugten Anwendung wird zunächst zur Durchführung des Verfahrens eine Probe 5 genommen. Bei der Probe 5 kann es sich zum Beispiel um eine Probe handeln, die von einem Patienten stammt, wie eine Oberflächenprobe von einem Patienten 16 und/oder eine Blutprobe und/oder Blutkultur und/oder Urinprobe.

Das Verfahren eignet sich besonders dazu, um einen Schnelltest auf das Vorliegen eines antibiotikaresistenten Keimes, insbesondere in einem Krankenhaus, bevorzugt vor Ort, durchzuführen. Insbesondere können durch das Verfahren auch das Vorhandensein multiresistenter Keime, wie multiresistenter gramnegative Bakterien, getestet werden.

Die Probe 5 wird anschließend in den Probenträger 17 eingeführt. Bei dem Probenträger 17 kann es sich beispielsweise um einen zur Mikrofluidik eingerichteten Probenträger handeln. Nun wird die Probe 5 in dem Probenträger 17 automatisch aufbereitet, wobei die Probe 5 mit einer spezifischen Substanz 3, wie einem profluoreszierenden antibiotikaähnlichen Farbstoff 12 kombiniert wird, die bevorzugt an der Außenseite der Mikroorganismen angebunden wird.

Der Begriff spezifische Substanz kann sich auf die Ähnlichkeit zu einem bestimmten Antibiotikum 18 beziehen, auf dessen Resistenz die Mikroorganismen getestet werden sollen und daher spezifisch durch ein Enzym 8 geschnitten wird. Dabei können die Mikroorganismen durch die Verwendung von mehreren spezifischen Substanzen auf multiple Resistenzen hin untersucht werden.

Anschließend wird die Nachweisreaktion durch die optische Signalerfassung 7 mittels Zytometrie durchgeführt. Die Nachweisreaktion kann auch mittels anderer optischer Verfahren, wie beispielsweise bildgebender Verfahren wie Fluoreszenzmikroskopie, durchgeführt werden. Beispielsweise kann die Nachweisreaktion auch an Mikrofluidik ausgeführt werden. Antibiotikaresistente Mikroorganismen exprimieren bestimmte Enzyme, die als Katalysatoren den Abbau und/oder die Inaktivierung von Antibiotika bewirken. Diese Enzyme interagieren mit der spezifischen Substanz 3 (Substrat; Farbstoff) und beispielsweise zersetzen diese. Dadurch kommt es zu einer Farbreaktion. Der zersetzte Farbstoff fluoresziert in der Nähe zum Mikroorganismus oder sogar im Mikroorganismus, wenn er aufgenommen wurde. Durch optische Auswertung 7, wie Zytometrie, in einem Zytometer 9, kann die Anzahl an fluoreszierenden und somit resistenten Mikroorganismen 15 bestimmt werden.

Aufgrund eines fehlenden Kultivierungsschrittes ist die Durchführung des Verfahrens 1 auch außerhalb eines Labors möglich, da die Gefahr von Kontaminationen sehr gering ist. Aus diesem Grund können die Proben 5 und/oder die zuvor genannten Tröpfchen 13, 14 nährmediumfrei sein.

Die Erfindung betrifft also insbesondere ein Nachweisverfahren 1 für antibiotikaresistente Mikroorganismen 2, wobei dabei wenigstens eine spezifische Substanz 3, die durch ein eine Antibiotikaresistenz bewirkendes Enzym 8 des Mikroorganismus 2 zersetzt wird, der Probe 5 zugegeben wird, wobei durch das vorhandene Enzym 8 eine Reaktion 4 ausgelöst wird, wobei bei der Reaktion 4 ein optisch nachweisbares Reaktionsprodukt 6 in der Nähe zum resistenten Mikroorganismus 2 erzeugt wird, das anschließend in einem optischen Nachweisverfahren 7 detektiert wird.

### Bezugszeichenliste

- 1: erfindungsgemäßes Verfahren
- 2: nachzuweisender Mikroorganismus
- 3: spezifische Substanz
- 4: eine Reaktion des nachzuweisenden Mikroorganismus 2
- 5: Probe mit Mikroorganismen
- 6: optisch nachweisbares Reaktionsprodukt
- 7: optische Detektion
- 8: spezifisches Enzym des Mikroorganismus 2
- 9: Zytometer
- 10: selbst-immobilisierende Substrate
- 11: radikales/reaktives Edukt, gebildet nach enzymatischer Umsetzung der selbst-immobilisierenden Substrate 10
- 12: Substrate, welche nach deren Umsetzung dissoziieren
- 13: Tröpfchen mit nachzuweisendem Mikroorganismus 2
- 14: Tröpfchen mit Nicht-nachzuweisendem Mikroorganismus
- 15: fluoreszierender nachzuweisender Mikroorganismus
- 16: Oberflächenprobe von einem Patienten
- 17: Probenträger
- 18: Antibiotikum

## Patentansprüche

1. Verfahren (1) zum Nachweis von Mikroorganismen (2) und/oder derer Eigenschaften, wobei wenigstens eine spezifische Substanz (3), die eine Reaktion (4) des nachzuweisenden Mikroorganismus (2) auslöst, einer Probe (5), in der die Mikroorganismen (2) nachgewiesen werden sollen, beigegeben wird, wobei bei der Reaktion (4) ein optisch nachweisbares Reaktionsprodukt (6) erzeugt wird, das anschließend in einem optischen Nachweisverfahren detektiert (7) wird, **dadurch gekennzeichnet, dass** das Reaktionsprodukt (6) bis zum optischen Nachweis in räumlicher Nähe zum reagierenden Mikroorganismus (2) gehalten wird.

2. Verfahren (1) nach dem vorangehenden Anspruch, **dadurch gekennzeichnet, dass** das Reaktionsprodukt (6) an eine Außenfläche des Mikroorganismus (2) anbindet.

3. Verfahren (1) nach einem der vorangegangenen Ansprüche, **dadurch gekennzeichnet, dass** die einzelnen Mikroorganismen von einer Phasengrenze vorzugsweise zu einem außen liegenden Trägerfluid, insbesondere zu einer vorzugsweise hydrophoben Flüssigkeit, umgeben werden, insbesondere wobei das Reaktionsprodukt (6) durch die Phasengrenzen eingeschlossen wird.

4. Verfahren (1) nach einem der vorangegangenen Ansprüche, **dadurch gekennzeichnet, dass** ein empfindlicher Bereich des Nachweisverfahrens auf eine Abmessung der Mikroorganismen eingestellt ist.

5. Verfahren (1) nach einem der vorangegangenen Ansprüche, **dadurch gekennzeichnet, dass** die oder eine die einzelnen Mikroorganismen umgebende Phasengrenze durch Einsprühen der Mikroorganismen in das oder ein Trägerfluid erzeugt wird.

6. Verfahren (1) nach einem der vorangegangenen Ansprüche, **dadurch gekennzeichnet, dass** das Reaktionsprodukt (6) auf einen Raum von weniger als 1000 µm Durchmesser beschränkt wird.

7. Verfahren (1) nach einem der vorangegangenen Ansprüche, **dadurch gekennzeichnet, dass** die Probe (5) nach Beigabe der wenigstens einen Substanz (3) erwärmt wird, insbesondere um mindestens 10°C oder mehr, und/oder dass das Reaktionsprodukt (6) in einer nährmittelfreien Umgebung und/oder kultivierungsfrei erzeugt wird.

8. Verfahren (1) nach einem der vorangegangenen Ansprüche, **dadurch gekennzeichnet, dass** als wenigstens eine spezifische Substanz (3) eine profluoreszierende und/oder eine prolumineszierende und/oder eine prophosphoreszierende Substanz und/oder eine Substanz, die einen Farbumschlag bewirkt, verwendet wird.

9. Verfahren (1) nach einem der vorangegangenen Ansprüche, **dadurch gekennzeichnet, dass** eine optische Nachweisgrenze so gesetzt ist, dass das Reaktionsprodukt (6) erst ab einer vorbestimmten Konzentration detektiert wird.

10. Scheibenförmiger Probenträger (17) mit Mitteln zum Ausführen eines Verfahrens (1) nach einem der vorangehenden Ansprüche, insbesondere mit einem Aufnahmeraum zur Abnahme der Mikroorganismen von einem Probennahmeinstrument und/oder einer Düse zum Einsprühen der Mikroorganismen in ein Trägerfluid und/oder mit einem Nachweisbereich zu einer quantitativen optischen Detektion (7) von Mikroorganismen (2), in deren räumlicher Nähe sich das Reaktionsprodukt (6) befindet und/ oder einem Filtermaterial zum Zurückhalten oder Konzentrieren der Mikroorganismen.
